# EUROPÄISCHE PATENTSCHRIFT

(11) **EP 0 389 517 B1**
(45) Veröffentlichungstag und Bekanntmachung des Hinweises auf die Patenterteilung: **19.01.1994**
(21) Anmeldenummer: 88909737.4
(22) Anmeldetag: 14.11.1988
(51) Int. Cl.: A61B 17/12, A44B 11/25

(54) **VORRICHTUNG ZUR BLUTSTAUUNG AN KÖRPERTEILEN**
DEVICE FOR LIGATURING PARTS OF THE BODY
DISPOSITIF HEMOSTATIQUE APPLICABLE A DES PARTIES DU CORPS

(30) Priorität: 17.11.1987 DE 3738903
(43) Veröffentlichungstag der Anmeldung: 03.10.1990
(73) Patentinhaber: STURM, Martina Elisabeth, D-51377 Leverkusen (DE)
(72) Erfinder: STURM, Martina Elisabeth, D-51377 Leverkusen (DE)
(74) Vertreter: Bauer, Wulf, Dr.
(86) Internationale Anmeldenummer: DE8800708
(87) Internationale Veröffentlichungsnummer: WO8904636

(56) Entgegenhaltungen:
- DE-A- 3 538 583
- US-A- 3 293 714

## Beschreibung

Die Erfindung bezieht sich auf eine Vorrichtung zur Blutstauung an Körperteilen nach dem Oberbegriff des Patentanspruchs 1.

Bei der aus der DE-OS 35 38 583 vorbekannten Vorrichtung der eingangs genannten Art sind die Verriegelungsmittel des Klemmteils als in den Seitenwangen vorgesehene, in Längsrichtung des Gurtes verlaufende, nach oben offene und einseitig geschlossene Ausnehmungen ausgeführt, die Verriegelungsmittel des Löseteils sind seitlich vorspringende Zapfen, die in diese Ausnehmungen eingreifen können. Beim Zusammensetzen der beiden Teile wird der Gurt lokal zwischen Klemmbereich und Grundfläche zusammengedrückt, die dabei entstehende elastische Kraft und ein Formschluß halten die beiden Teile im zusammengesetzten Zustand. Bei dieser vorbekannten Vorrichtung ist ein sanfter Entstauvorgang, wie er beispielsweise nach einer Injektion erforderlich sein kann, nicht einfach zu bewirken. Der vom Schwenkgelenk gesehen von der Schlinge abgewandte Hebelarm, der die Klemmwirkung erzeugt, ist im wesentlichen gleichlang wie der andere Hebelarm, also der vom Schwenkgelenk gesehen der Schlinge zugewandte Bereich des Löseteils. Über letzteren Hebelarm wird die von der Schlinge bewirkte, die beiden Teile in Richtung des Verlaufs des Gurtes im Bereich der Schlinge auseinanderziehende Kraft eingeleitet. Aufgrund der Hebelverhältnisse bedarf es nur einer kleinen Bewegung des der Schlinge zugewandten Bereichs des Löseteils, um ein schlagartiges Freigehen des Gurtes zu erreichen. Diesem Effekt kann man durch quer zur Gurtzugrichtung angeordnete Rippen oder Kanten im Klemmbereich entgegenwirken, die die Reibung zwischen Gurt und Klemmbereich vergrößern. Sie bewirken aber einen starken Gurtverschleiß, die Entstaugeschwindigkeit läßt sich nicht präzise vorgeben. Der für ein Zusammenschiehen von Löseteil und Klemmteil benötigte Schubweg ist relativ lang.

Weiterhin ist aus dem DE-GM 75 29 390 ein Gurtstauer bekannt, dieser hat ebenfalls die genannten Nachteile.

Ausgehend von der Vorrichtung der eingangs genannten Art liegt der Erfindung die Aufgabe zugrunde, diese Vorrichtung dahingehend weiterzubilden, daß der Entstauvorgang sanft und kontrolliert ausgelöst werden kann, der Staudruck auch im Gurteinzugsbereich erzeugt werden kann, das Lösen der Teile vereinfacht wird und durch die Teilegestaltung eine kostengünstige Ausführungsform erreicht wird.

Diese Aufgabe wird gelöst durch die Vorrichtung mit den Merkmalen des Patentanspruchs 1.

Diese Konstruktion ermöglicht es, daß der Klemmbereich in unmittelbarer Nähe des Schwenkgelenks ist, wodurch der entsprechende Hebelarm relativ kurz wird. Dies hat zur Folge, daß der andere Hebelarm des Löseteils, dessen Länge durch den Abstand zwischen Schwenkgelenk und vorderer Außenfläche bestimmt ist, eine wesentlich größere Länge als der erste Hebelarm haben kann, beispielsweise die zweifache bis dreifache Länge. Weiterhin lassen sich Klemmteil und Löseteil als weitgehend formgleiche, vorzugsweise vollständig formgleiche Teile ausbilden, dies senkt die Herstellungskosten. Der Verbindungsbereich und damit das Schwenkgelenk zwischen den beiden Teilen ist als brückenartige Konstruktion ausgelegt, die zur Mitte des jeweiligen Teils weisende Brückenseite ist als trapezförmige Nase ausgebildet und der sich darunter befindende Einsteckbereich weist eine, um 180 Grad quer zur Längsachse gedrehte, gleichförmige Hohlform auf. Die trapezförmige Gestaltung der Nase und des Aufnahmebereichs sichern die Teile gegen seitliches Verschieben. Dies wird noch dadurch unterstützt, daß Löseteil und Klemmteil Seitenwangen haben, die sich etwa über die halbe Gesamtlänge jedes Teils erstrecken und soweit vorspringen, daß im zusammengesetzten Zustand ein Teilstück eines Grundkörpers des jeweils andere Teil sich zwischen den freien Enden der Seitenwangen befindet.

Im locker zusammengesteckten Zustand von Klemmteil und Löseteil, also ohne Spannung innerhalb der Schlinge, wird der zusammengesetzte Zustand der Gurtschnalle entweder dadurch aufrecht erhalten, daß die Kanten gegenüber den restlichen Flächen der Brückenteile vorspringen und beim Zusammenschieben der beiden Teile einander formschlüssig hintergreifen, wobei der Abstand zwischen Klemmbereich des Löseteils und Grundfläche des Klemmteils so gewählt ist, daß beim Übereinandergleiten der beiden Kanten (also während des Zusammenschiebens oder Lösens) der Gurt zusammengedrückt wird, im zusammengesetzten Zustand der Gurtschnalle aber dieser Abstand zwischen Grundfläche und Klemmbereich so ausreichend groß bemessen ist, daß ein Festklemmen des Gurtes nicht stattfindet, vielmehr der Gurt innerhalb der Gurtschnalle noch bewegt werden kann, um die Schlaufe zu verändern. Er wird dann erst festgeklemmt, wenn ein Zug in der Schlaufe auftritt. Entsprechend diesen Gegebenheiten ist die Höhe der als Vorsprünge ausgebildeten Kanten zu bemessen, sie liegen wenige Zehntel Millimeter, beispielsweise 0,2 bis 0,3 mm, der Grundfläche des jeweils zugehörigen Grundkörpers näher als die übrigen Teilbereiche des Brückenteils oder der Zusammenhalt der Gurtschnalle wird nicht formschlüssig, sondern ausschließlich reibschlüssig bewirkt, wobei vorspringende Kanten nicht notwendig sind, vielmehr der Abstand der Grundfläche vom Klemmbereich so bemessen ist, daß auch im nicht gespannten Zustand der Schlaufe der Gurt im Klemmbereich geringfügig zusammengedrückt ist, wodurch die notwendige elastische Kraft erhalten wird, um die Kanten gegeneinander zu drücken. Das notwendige Einklemmen des Gurtes muß aber so sein, daß der Gurt trotzdem noch durch die Gurtschnalle bewegt werden kann, um die Schlaufe größenmäßig verändern zu können. Zwischen den Extremen eines rein formschlüssigen bzw. eines rein reibschlüssigen Haltes der beiden Teile der Gurtschnalle im kraftlosen Zustand der Schlaufe sind alle zwischenliegenden Übergangsformen möglich. Die der Verhakung der beiden Teile dienenden Kanten sind an einem Profil mit dachförmigem Überstand ausgebildet, das Teil des Brückenteils ist. Unter dem Brückenteil befindet sich eine Kammer, durch die der Gurt in dem Klemmteil geführt ist. Er verläuft dabei über eine Grundfläche. Am schlaufenseitigen Endbereich des Klemmteils springt von der Grundfläche eine Querrippe vor, zusammen mit dem Brückenteil begrenzt ihre Oberkante eine rechteckige Öffnung, durch die der Gurt geführt ist. Die Querrippe erzeugt eine gurtschonende Gleitreibung zwischen dem gleitenden Gurt und dem Klemmteil während des Entstauens und sichert einen sanften Entstauvorgang.

Die beiden Teile des Gurtschlosses sind gegeneinander um die Kanten kippbar, die Kanten bilden also zwei Achsen eines Schwenkgelenks. Im zusammengesetzten Zustand liegen die Kanten sehr eng beieinander, ihr Abstand liegt vorzugsweise unter 2 mm. Dementsprechend ist auch der Weg, um den die beiden Teile beim Zusammenschieben gegeneinander oder beim Lösen voneinander bewegt werden müssen, äußerst kurz. Dies ist ein großer Vorteil in der praktischen Handhabung.

Die Kanten erstrecken sich quer zum Gurt nur etwa über einen Teil der Gurtbreite, beispielsweise 1/3 bis 2/3 der Gurtbreite, vorzugsweise 50 % der Gurtbreite.

In vorzugsweiser Ausbildung ist der Klemmbereich so gestaltet, daß in der entspannten Position der Schlaufe die vordere Außenfläche des Löseteils zu einer hinteren Außenfläche des Klemmteils um vorzugsweise 5 Grad zur (entspannten) Schlinge hin geneigt ist und die vordere Außenfläche des Klemmteils durch Formgestaltung dieses Klemmteils um vorzugsweise 5 Grad zur Schlinge hin angehoben ist. Die hintere Außenfläche des Klemmteils steht zur hinteren Außenfläche des Löseteils parallel.

Der Abstand einer vorderen Außenfläche des Klemmteils von den Kanten ist mindestens doppelt so groß wie der Abstand zwischen Kanten und Klemmbereich. Jedes Teil besteht in einer vorteilhaften Weiterbildung aus einem Grundkörper, zwei Seitenwangen und einem Brückenteil. Die seitlich am Grundkörper angesetzten Seitenwangen erstrecken sich etwa über die halbe Gesamtlänge des Grundkörpers und haben einen Abstand, der größer ist als die Gurtbreite und der ausreicht, den Grundkörper des jeweils anderen Teils zwischen sich aufzunehmen. Dementsprechend springen die Seitenwangen auch soweit vor, daß im zusammengesetzten Zustand der Gurtschnalle sich der Grundkörper des anderen Teils zwischen ihnen befindet. Hierdurch wird vermieden, daß die beiden zusammengesetzten Teile seitlich gegeneinander verschoben werden können. Die Seitenwangen bilden mit ihren der Schlaufe zugewandten Stirnflächen vorteilhafterweise den der Schlaufe am nächsten liegenden Teil des Gurtschlosses. Vorzugsweise liegen diese Stirnflächen mehrere Millimeter vor der rechteckförmigen Öffnung für den Einlauf des Gurtes im Klemmteil. Dadurch wird der von der Schlaufe ausgesparte Teil eines Vollkreises durch die beiden Stirnflächen der Seitenwangen des Klemmteils überbrückt, die Stirnflächen wirken als Preßfläche und sichern eine vollständig geschlossene Stauung über den gesamten Umfang.

In einer weiteren vorteilhaften Ausbildung ist eine Blutdruckmeßvorrichtung an der Innenseite der Schlaufe angeordnet, hierdurch wird die Vorrichtung zur Einrichtung für Blutdruckmessung erweitert.

Weitere Vorteile und Merkmale der Erfindung ergeben sich aus den übrigen Ansprüchen sowie der nun folgenden Beschreibung eines nicht einschränkend zu verstehenden Ausführungsbeispiels, das unter Bezugnahme auf die Zeichnung näher erläutert wird. In dieser zeigen:
- Fig. 1: Eine Seitenansicht der gesamten Vorrichtung,
- Fig. 2: ein Schnitt bei einer Ansicht entsprechend Figur 1 durch den Bereich der Gurtschnalle,
- Fig. 3: eine Draufsicht auf den Bereich der Gurtschnalle,
- Fig. 4: eine stirnseitige Ansicht auf die Gurtschnalle bei geschnittenem Gurt,
- Fig. 5: eine Ansicht entsprechend Figur 3, jedoch ausschließlich auf das Klemmteil und
- Fig. 6: eine schematische Darstellung zur Erläuterung der Hebelverhältnisse und der Kraftverteilung im Sinne einer Darstellung entsprechend Figur 1.

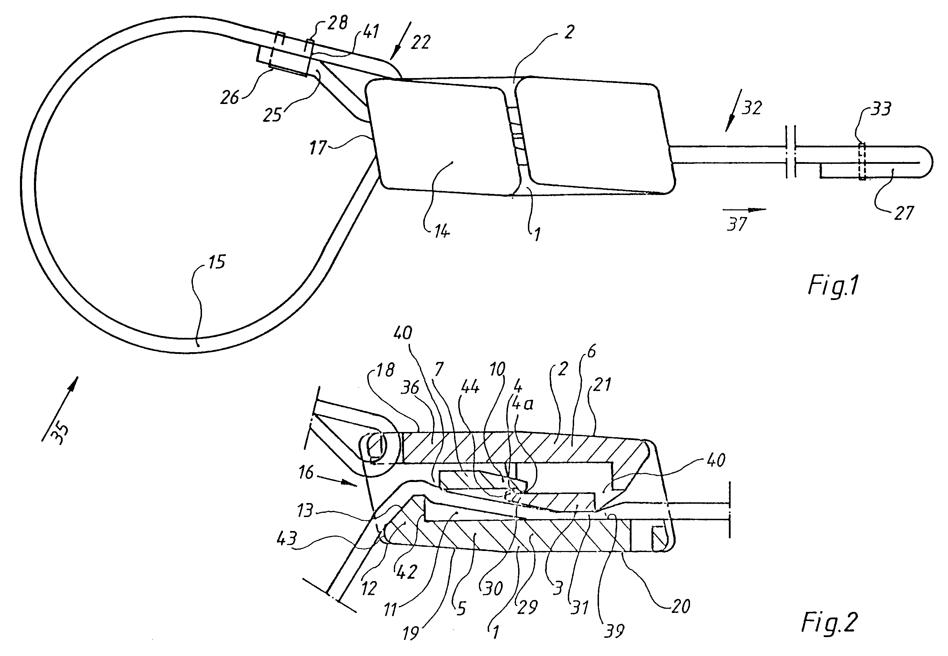

Die Darstellungen der Figuren 1 bis 5 zeigen die Vorrichtung in gespannter Gebrauchsposition, in der in der Schlaufe bzw. Schlinge eine Zugspannung besteht.

Die Vorrichtung besteht aus einem, mit einem Teil seiner Gesamtlänge eine Schlinge 35 bildenden Gurt 15, einem Klemmteil 1 und einem Löseteil 2. Im gezeigten Ausführungsbeispiel sind Klemmteil 1 und Löseteil 2 geometrisch gleiche, also baugleiche Teile, sie können durch Längsverschiebung in Längsrichtung des Gurtes 15 miteinander verhakt und voneinander gelöst werden. Bei beiden Teilen 1, 2 ist jeweils auf einem Grundkörper 29 eine brückenartige Konstruktion angeordnet, die aus jeweils zwei Seitenwangen 14 und einem diese verbindenden Brückenteil 7 besteht. Letzteres hat auf der einen Seite einen dachförmigen, angeschrägten Überstand 10. Zwischen dem Brückenteil 7 und einer Grundfläche 39 des Grundkörpers 29 wird eine Kammer 11 ausgebildet, der Gurt 15 verläuft durch die Kammer 11 des Klemmteils 1. Die Kammer wird zur Schlinge 35 hin durch eine Querrippe 12 abgeschlossen, die quer zur Grundfläche 39 aufragt. Ihre Oberkante liegt mehrere Millimeter, beispielsweise 2 bis 3 mm oberhalb der Grundfläche 39. Mit einer unteren Hinterkante des Brückenteils 7 des Klemmteils 1 begrenzt sie eine rechteckige Öffnung 40, durch die der Gurt 15 hindurchläuft. Die Querrippe hat zur Seite der Schlinge 35 hin eine Schrägfläche 13, die in einem Winkel von 30 bis 60 Grad, vorzugsweise 45 Grad zur Grundfläche 39 steht. Diese befindet sich, wie insbesondere Fig. 2 zeigt, inseits von Stirnflächen 17 der Seitenwangen 14, eine abgerundete Übergangskante 43 liegt wenige Zehntel Millimeter gegenüber der Stirnfläche 17 zurück, die Oberkante der Querrippe 12 liegt etwa 5 bis 6 mm hinter dieser Stirnfläche 17 zurück. Die Stirnfläche 17 verläuft in einem Winkel größer 90 Grad zur Grundfläche 39, beispielsweise im Winkel von 100 Grad. Die Querrippe 12 befindet sich zwischen den Seitenteilen 14.

Mit dem Bezugszeichen 16 ist ein Gurteinzugsbereich bezeichnet, dort ist, wie insbesondere Figur 2 zeigt, ein Freiraum auf der vom Klemmteil 1 abgewandten Seite des Gurtes 15, der etwas breiter ist als die Gurtbreite und insbesondere nach einer Engstelle sich wieder erweitert, also keine trichterförmige Zuspitzung hat.
Das Löseteil 2 ist fest mit einem Gurtende 22 verbunden, das eine Schlaufe bildet. Hierzu ist eine Öffnung 23 im Grundkörper 29 des Löseteils 2 vorgesehen, die durch einen Quersteg 24 zur Schlaufe hin begrenzt ist. Ein überlappendes Gurtteil 25 wird durch ein Klammerelement 26 mit dem Gurt 15 verbunden, wodurch die erwähnte Befestigungsschlaufe gebildet wird, die Enden des Klammerelementes 26 sind in U-Form 28 abgewinkelt, um Verletzungen zu vermeiden.

Das Material des Gurtes 15 ist längs- und querelastisch. Der Gurt hat typischerweise eine Breite von 23 mm und eine Dicke von 2,5 mm bei rechteckförmigen Querschnitt. Eine vordere Außenfläche 18 des Löseteils 1 bzw. eine hintere Außenfläche 20 des Klemmteils 2 verläuft jeweils parallel zur zugehörigen Grundfläche 39. Eine vordere Außenfläche 19 des Klemmteils 1 und auch eine hintere Außenfläche 21 des Löseteils 2 verlaufen hierzu jeweils leicht geneigt, gezeigt ist ein Winkel von etwa 5 Grad. Die Seitenwangen 14 haben Parallelogrammform, wie insbesondere aus Fig. 1 hervorgeht. Die Flächen 18 bis 21 haben jeweils etwa gleiche Länge.

In Längsrichtung des Gurtes 15 beträgt die Lange der Brückenteile 7 etwa 1/3 der Gesamtlänge jedes Teils 1 bis 2. Im Bereich der rechteckförmigen Öffnung 40 des Klemmteils 1 drückt eine untere Kante des Brückenteils 7 den Gurt geringfügig nach unten, so daß ein leicht S-förmiger Verlauf, wie er aus Fig. 2 ersichtlich ist, entsteht. Hierdurch wird eine Reibung, die jedoch den Gurt 15 schont, erzielt. Zugleich ist auch der Klemmteil 1 unverlierbar mit dem Gurt 15 verbunden, hierzu ist ein hinteres Gurtende bei 27 überlappt und mittels einer Verklammerung 33 so verdoppelt, daß eine Dicke entsteht, die deutlich größer ist als die lichte Weite der Öffnung 40. Aufgrund der Elastizität kann jedoch mit etwas Gewalt das verdickte Ende durch die Öffnung 40 hindurchbewegt werden, so daß sich der Klemmteil 1 vom Gurt 15 lösen läßt.

Bei separierten Teilen 1, 2 und gestreckt ausgelegtem Gurt 15 liegen die beiden Teile 1, 2 gleichgerichtet und nur in Gurtrichtung versetzt hintereinander, die Schlaufe 35 wird dadurch gebildet, daß ein Teil auf das andere geschlagen wird, wodurch die beiden Teile 1, 2 die in den Figuren 1 bis 4 gezeigte Position einnehmen, sobald Spannung in der Schlinge 35 herrscht. Im schlaffen Zustand der Schlinge 35 ist der Gurt im Bereich der lichten Öffnung 31 nicht zusammengequetscht, wie in Fig. 2 dargestellt, sondern hat seine normale Dicke, so daß er in Richtung des Pfeiles 37 verschoben werden kann.

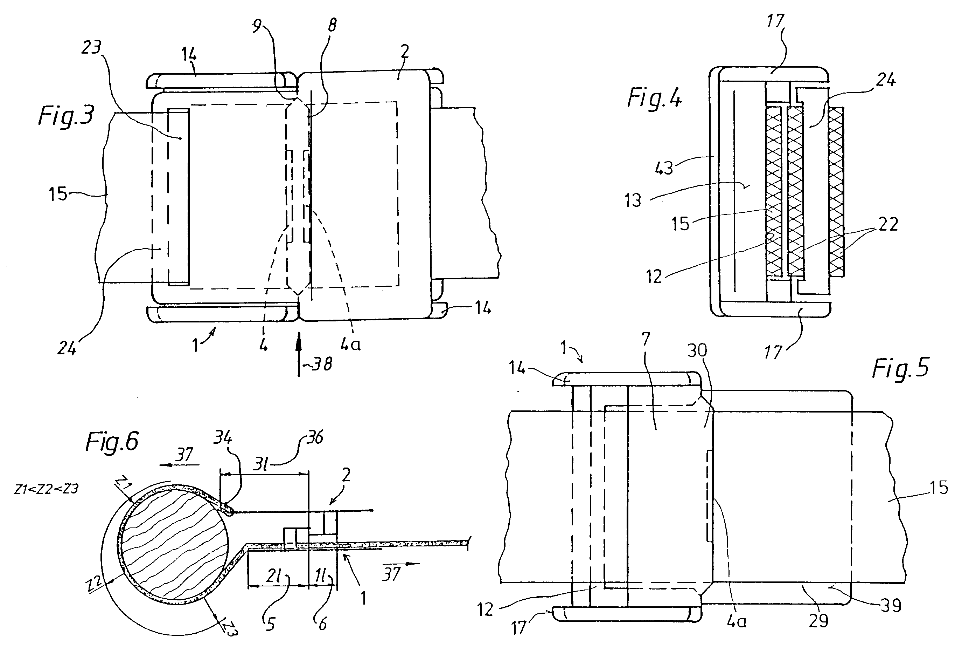

Figur 6 zeigt in schematischer Darstellung den praktischen Einsatz. Der Gurt 15 wird in Form der Schlinge 35 um das abzubindende Körperteil gelegt, das Löseteil 2 wird mit dem Klemmteil 1 durch Längsverschiebung um wenige Millimeter verhakt. In Richtung des Pfeils 37 wird der Gurt 15 durch Längszug am freien Gurtende 32 gespannt, dadurch entsteht eine Spannung in der Schlinge 35. Die Gurtspannung steigt beginnend an der Anschlaufung 34 im Verlauf der Schlinge bis zum Einlauf in das Klemmteil 1 an, dies ist durch die entsprechend länger werdenden Pfeile Z1, Z2 und Z3 dargestellt. Durch die Gurtspannung wird eine Kraft bewirkt, die die der Schlinge 35 zugewandten Endbereiche der beiden Teile 1, 2 voneinander separiert, also bestrebt ist, den Gurteinzugsbereich 16 zu öffnen. Sie bewirkt über die Gelenkverbindung der beiden Kanten 4, 4a und der Auflageflächen des Brückenteils 7, auf denen diese Kanten 4, 4a aufliegen, eine Einschnürung des Gurtes im Bereich der lichten Öffnung 31, wie aus Fig. 2 ersichtlich ist. Aus Fig. 6 sind auch die Hebelverhältnisse im Bereich der Gurtschnalle ersichtlich. Danach beträgt der Abstand 36 zwischen den Kanten 4, 4a und der Anschlaufung 34 3 l, der Abstand zwischen Stirnfläche 17 und den Kanten 4, 4a ist mit 5 bezeichnet, er ist dar lange Hebel und beträgt 2 l, dar Abstand der Kanten 4, 4a von der rückwärtigen Kante des Klemmbereichs 3 ist mit 6 bezeichnet, er ist der kurze Hebel und beträgt 1 l. Das Klemmteil 1 bildet somit einen zweiarmigen Hebel, über den langen Hebelarm 5 wird die Zugkraft eingeleitet und über den kurzen Hebelarm 6 die Klemmung bewirkt. Die Länge des Lösehebels 36 ist deutlich größer als die Länge des kurzen Hebels, so daß ein sehr gut dosiertes Entstauen erreicht wird. Die Gurtzugskraft Z baut sich im Verlauf der Umschlingung des Körperteils ab, so daß beim Lösen des Löseteils nur die Restzugkraft Z1 entgegenwirkt. Auch hierdurch wird das Lösen besser dosierbar.

Das Löseteil 2 ist nach Aufheben der Gurtzugkraft durch leichtes Zurückziehen des dachförmigen Überstandes 10, dies bedeutet eine Strecke von wenigen Millimetern, des Löseteils zwei aus der Kammer des Klemmteils 1 trennbar.

Die Fläche 18 des Löseteils 2 bildet im entspannten Zustand der Gurtschnalle mit der Fläche 19 des Klemmteils 1 einen zur Gurtschlinge 35 geneigten Keil zur Unterstützung der Handhabung beim Entstauen, Lösen und Trennen.

## Patentansprüche

1. Vorrichtung zur Blutstauung an Körperteilen mit einem eine Schlaufe (35) bildenden, flachen Gurt (15) aus einem elastischen Material und einer an diesem befestigten Gurtschnalle, die aus einem Klemmteil (1) und einem Löseteil (2) besteht, wobei das Klemmteil einen Grundkörper (29) aufweist, der eine Grundfläche (39) für die Auflage und Führung in Längsrichtung des Gurtes (15) ausbildet und Seitenwangen hat, die Verriegelungsmittel für eine lösbare Verbindung von Klemmteil (1) und Löseteil (2) tragen und zwischen denen der Gurt (15) verläuft, und das Löseteil (2) mit einem Gurtende (22) verbunden ist, Verriegelungsmittel für die lösbare Verbindung mit dem Klemmteil (1) hat und an seinem von der Schlinge (35) abgewandten Endbereich einen Klemmbereich (3) aufweist, dessen Abstand von der Grundfläche (39) im zusammengesetzten Zustand der Gurtschnalle im Bereich der Dicke des entspannten Gurtes (15) liegt und die Verriegelungsmittel im zusammengesetzten Zustand der Gurtschnalle ein Schwenkgelenk bilden und bei einem Druck auf eine vordere Außenfläche (18) des Löseteils (2), die sich zwischen den Verriegelungsmitteln und der Schlinge (35) befindet, der Klemmbereich (3) von der Grundfläche (39) entfernt wird und dadurch die Klemmwirkung aufgehoben wird,
dadurch gekennzeichnet, daß Klemmteil (1) und Löseteil (2) jeweils ein Brückenteil (7) aufweisen, das quer zum Gurt (15) verläuft, daß das Brückenteil (10) des Klemmteils (1) an seinem von der Schlinge (35) abgewandten Endbereich eine zur Grundfläche (39) weisende und quer zum Gurt (15) verlaufende Kante (4a) und das Löseteil am entgegengesetzten Endbereich eine von der Grundfläche (39) wegweisende, quer zum Gurt verlaufende entsprechende Kante (4) aufweist, daß diese Kanten (4, 4a) im zusammengesetzten Zustand der Gurtschnalle nebeneinander liegen, das Schwenkgelenk ausbilden und sich auf der von der Grundfläche (39) abgewandten Seite des Gurtes (15) befinden.

2. Vorrichtung nach Anspruch 1, dadurch gekennzeichnet, daß an den Kanten (4, 4a) Verdickungen (44) angeordnet sind.

3. Vorrichtung nach Anspruch 1 oder 2, dadurch gekennzeichnet, daß Klemmteil (1) und Löseteil (2) baugleich ausgebildete Brückenteile (7) aufweisen.

4. Vorrichtung nach einem der Ansprüche 1 bis 3, dadurch gekennzeichnet, daß sich die Kanten (4, 4a) mindestens über die halbe Breite des Gurtes (15) erstrecken.

5. Vorrichtung nach einem der Ansprüche 1 bis 4, dadurch gekennzeichnet, daß Klemmteil (1) und Löseteil (2) baugleich ausgebildet sind.

6. Vorrichtung nach einem der Ansprüche 1 bis 5, dadurch gekennzeichnet, daß die Kanten (4, 4a) etwa in der Querschnittsmitte der zusammengesetzten Gurtschnalle verlaufen.

7. Vorrichtung nach einem der Ansprüche 1 bis 6, dadurch gekennzeichnet, daß der Abstand der Kanten (4, 4a) vom Klemmbereich (3) kleiner ist als ihr Abstand von einer vorderen Außenfläche (18), insbesondere mindestens um den Faktor zwei kleiner ist.

8. Vorrichtung nach einem der Ansprüche 1 bis 7, dadurch gekennzeichnet, daß auch das Löseteil (2) Seitenwangen (14) hat und daß die Seitenwangen (14) sowohl des Löseteils (2) als auch des Klemmteils (1) jeweils durch ein Brückenteil (7) miteinander verbunden sind, wodurch zwischen Grundfläche (39) und Brückenteil (7) des Klemmteils (1) eine Öffnung (40) ausgebildet wird, durch die der Gurt (15) hindurchgeführt ist.

9. Vorrichtung nach Anspruch 8, dadurch gekennzeichnet, daß die lichte Höhe der Öffnung (40) größer ist als die einfache Dicke, aber kleiner ist als die doppelte Dicke des Gurtes (15).

10. Vorrichtung nach einem der Ansprüche 1 bis 9, dadurch gekennzeichnet, daß das Löseteil (2) ein Langloch (23) für die Aufnahme einer Halteschlaufe des Gurtes (15) hat.

11. Vorrichtung nach einem der Ansprüche 1 bis 10, dadurch gekennzeichnet, daß am der Schlinge (35) zugewandten Endbereich der Grundfläche (39) eine Querrippe (12) in Richtung zum Löseteil (2) vorspringt, die vorzugweise eine Schrägfläche (13) für die Gurtauflage aufweist.

## Claims

1. Device for stopping the flow of blood in extremities consisting of a flat strap (15) made of an elastic material that forms a loop (35) and of a strap-clamping device that is attached to the strap and that consists of a clamping part (1) and a release part (2); the clamping part has a foundation (29) that forms a base (39) for supporting and guiding the strap (15) in the longitudinal direction and that has side walls, which have first locking mechanisms for releasable fastening of the clamping part (1) and the release part (2), and between which the strap (15) passes; the release part (2) is attached to one end (22) of the strap, has second locking mechanisms for releasable fastening with the clamping part (1) and has a clamping zone (3) at the far end of the release part away from the loop (35); the distance of the clamping zone from the base (39) in the assembled state of the strap-clamping device is approximately the same as the thickness of the loose strap (15); in the assembled state of the strap clamping device the first and second locking mechanisms form a swivel joint; which is located between the first and second locking mechanisms and the loop (35), and when a forward outer surface (18) of the release part (2) is pressed, the clamping zone (3) is separated from the base (39), thereby releasing the clamping action; characterized in that the clamping part (1) and the release part (2), respectively, have a bridge part (7), which runs traverse to the strap (15), that an edge (4a) is arranged on the bridge part (7) of the clamping part (1), is located at it's end opposite to the loop (35), which points toward the base (39) and runs traverse to the strap (15), that a similar edge (40) is arranged on the release part (2) at the opposite end thereof, points away from the base (39) and runs traverse to the strap (15), that these edges (4, 4a) in the assembled state of the strap-clamping device are positioned side by side, form the swivel joint and are located at the side of the strap (15) locking away from the base (39).

2. Device in accordance with claim 1, characterized in that enlargements (44) are provided at the edges (4, 4a).

3. Device in accordance with claims 1 or 2, characterized in that the clamping part (1) and the release part (2) have identical bridge parts (7).

4. Device in accordance with one of the claims 1 to 3, characterized in that the edges (4, 4a) extend over at least 50 % of the width of the strap (15).

5. Device in accordance with one of the claims 1 to 4, characterized in that the clamping part (1) and the release part (2) are identical parts.

6. Device in accordance with one of the claims 1 to 5, characterized in that the edges (4, 4a) run approximately in the middle of the section of the assembled strap-clamping device.

7. Device in accordance with one of the claims 1 to 6, characterized in that the distance between the edges (4, 4a) and the clamping zone (3) is smaller than the distance between the edges (4, 4a) and a forward outer surface (18), especially is smaller by a factor of two.

8. Device in accordance with one of the claims 1 to 7, characterized in that the release part (2), too, has side walls (14) and that the side walls (14) of the release part (1) and those of the clamping part (1) are mutually connected by a bridge part (7), so that there is an opening (40) between the base (39) and the bridge part (7) of the clamping part (1) for the passage of the strap (15).

9. Device in accordance with claim 8, characterized in that the free height of the opening (40) is more than one times the thickness but less than two times the thickness of the strap (15).

10. Device in accordance with one of the claims 1 to 9, characterized in that the release part (2) has an oblong hole (23) for receiving a fixation loop of the strap (15).

11. Device in accordance with one of the claims 1 to 10, characterized in that a traverse rib (12) projects from the base (39) at the end facing the loop (35) and in direction to the release part (2), which preferably has an inclined surface (13) supporting the strap (15).

## Revendications

1. Dispositif hémostatique applicable à des parties du corps avec une ceinture plate (15) sous la forme d'une courroie (35), en matériau élastique, sur laquelle est fixée une boucle constituée d'un élément de blocage (1) et d'un élément de desserrage (2); l'élément de blocage présente une partie de base (29) avec une surface (39) d'appui et de guidage de la ceinture (15) dans le sens longitudinal, et qui comprend des parois latérales, qui supportent les éléments de verrouillage de la liaison amovible de l'élément de blocage (1) et de l'élément de desserrage (2), entre lesquelles passe la ceinture (15) et où l'élément de desserrage (2) est relié à une extrémité de la ceinture (22), possède des éléments de verrouillage pour la liaison amovible de l'élément de blocage (1) et présente à l'extrémité opposée à la boucle (35) une plage de blocage (3), dont l'intervalle par rapport à la surface (39), lorsque la boucle de la ceinture est fermée, se trouve dans la zone de l'épaisseur de la ceinture desserrée (15) et les éléments de verrouillage, lorsque la boucle de la ceinture est fermée, forment un joint articulé, et en cas de pression sur une surface extérieure avant (18) de l'élément de desserrage (2), surface qui se trouve entre les éléments de verrouillage et la boucle (35), la plage de blocage (3) est enlevée de la surface (39) et ainsi l'effet de blocage est supprimé,
caractérisé par le fait que l'élément de blocage (1) et l'élément de desserrage (2) présentent chacun une pièce de jonction (7), placée transversalement à la ceinture (15), que la pièce de jonction (10) de l'élément de blocage (1) présente une arête (4a) transversale à la ceinture, à son extrémité opposée à la boucle (35), du côté de la surface (39), et que l'élément de desserrage présente une arête (4) correspondante, transversale à la ceinture, à l'extrémité opposée, dans le sens opposé à la surface (39), que ces arêtes (4, 4a) sont l'une à côté de l'autre lorsque la boucle de la ceinture est fermée, qu'elles forment le joint articulé et se trouvent sur le côté de la boucle (15) opposé à la surface (39).

2. Dispositif selon la sollicitation 1, caractérisé par le fait que des épaisseurs (44) se trouvent sur les arêtes (4, 4a).

3. Dispositif selon les sollicitations 1 ou 2, caractérisé par le fait que l'élément de blocage (1) et l'élément de desserrage (2) présentent des pièces de jonction (7) constituées de la même manière.

4. Dispositif selon une des sollicitations 1 à 3, caractérisé par le fait que les arêtes (4, 4a) s'étendent au moins sur la moitié de la largeur de la ceinture (15).

5. Dispositif selon une des sollicitations 1 à 4, caractérisé par le fait que l'élément de blocage (1) et l'élément de desserrage (2) sont constitués de la même manière.

6. Dispositif selon une des sollicitations 1 à 5, caractérisé par le fait que les arêtes (4, 4a) passent à peu près au milieu de la coupe transversale de la boucle fermée de la ceinture.

7. Dispositif selon une des sollicitations 1 à 6, caractérisé par le fait que l'intervalle entre les arêtes (4, 4a) et la plage de blocage (3) est plus petit que l'intervalle entre les arêtes (4, 4a) et l'une des surfaces extérieures avant (18); plus petit au moins d'un facteur deux.

8. Dispositif selon une des sollicitations 1 à 7, caractérisé par le fait que l'élément de desserrage (2) a lui aussi des parois latérales (14) et que les parois (14) de l'élément de desserrage (2) aussi bien que celles de l'élément de blocage (1) sont reliées par une pièce de jonction (7), ce qui forme une ouverture (40) pour le passage de la ceinture (15), entre la surface (39) et la pièce de jonction (7) de la pièce de blocage.

9. Dispositif selon la sollicitations 8, caractérisé par le fait que le jour de l'ouverture (40) est plus grand que l'épaisseur simple de la ceinture (15), mais plus petit que la double épaisseur.

10. Dispositif selon une des sollicitations 1 à 9, caractérisé par le fait que l'élément de desserrage (2) comprend un trou oblong (23) pour le logement d'une courroie de soutien de la ceinture (15).

11. Dispositif selon une des sollicitations 1 à 10, caractérisé par le fait qu'à l'extrémité de la surface (39), du côté de la boucle (35), une nervure transversale (12) dépasse en direction de l'élément de desserrage (2); cette nervure comprend une surface oblique (13) pour le support de la ceinture.
